# EUROPÄISCHE PATENTSCHRIFT

(11) **EP 0 745 355 B1**
(45) Veröffentlichungstag und Bekanntmachung des Hinweises auf die Patenterteilung: **09.09.1998**
(21) Anmeldenummer: 96102643.2
(22) Anmeldetag: 22.02.1996
(51) Int. Cl.: A61B 17/39

(54) **Zangen- oder scherenförmiges chirurgisches Instrument**
Forceps- or scissor-like surgical instrument
Instrument chirurgical en forme de pince ou de ciseaux

(30) Priorität: 02.05.1995 DE 19515914
(43) Veröffentlichungstag der Anmeldung: 04.12.1996
(73) Patentinhaber: AESCULAP AG, 78532 Tuttlingen (DE)
(72) Erfinder: Schwartz Theodor, D-78532 Tuttlingen (DE); Dworschak Manfred, D-78532 Tuttlingen (DE)
(74) Vertreter: Böhme, Ulrich

(56) Entgegenhaltungen:
- EP-A- 0 517 244
- EP-A- 0 584 787
- US-A- 4 370 980
- US-A- 5 026 370
- US-A- 5 116 332
- US-A- 5 380 320

## Beschreibung

Die Erfindung betrifft ein zangen- oder scherenförmiges chirurgisches Instrument mit zwei gegeneinander schwenkbaren Branchen, die an einer Lagerstelle drehbar miteinander verbunden sind und in diesem Bereich unter Ausbildung einer elektrischen Kontaktstelle an der Innenseite der Branchen aneinander anliegen.

Ein gattungsgemäßes Instrument ist in US-A-5 116 332 offenbart. Weiterhin offenbart US-A-5 026 370 eine elektrochirurgische Zange, bei der beide Branchen mit einer Ummantelung aus einem elektrisch isolierenden Material versehen sind, und bei der an einer Branche ein Anschluß für eine elektrische Verbindungsleitung und an beiden Branchen an deren freiem Ende eine Gewebeanlagefläche freibleibt.

Zangen- oder scherenförmige chirurgische Instrumente dieser Art werden zum Fassen von Gewebe oder auch zum Schneiden verwendet. Die Instrumente werden als eine Elektrode eines elektrischen Koagulationssystems eingesetzt, dazu wird das metallisch ausgebildete Instrument mit einer elektrischen Verbindungsleitung verbunden, die andere Elektrode des Systems wird durch den Körper selbst gebildet, der über eine Anlageelektrode ebenfalls mit einer elektrischen Leitung verbunden wird.

Bei derartigen Instrumenten ist es wesentlich, daß ein elektrischer Kontakt zwischen dem Instrument einerseits und dem Gewebe andererseits möglichst nur in den Bereichen stattfindet, in denen eine Koagulation erfolgen soll, andererseits muß sichergestellt werden, daß das gesamte Instrument im Gewebeanlagebereich als Elektrode wirkt, obwohl üblicherweise nur eine der beiden Branchen des Instrumentes mit der elektrischen Verbindungsleitung verbunden wird.

Es ist Aufgabe der Erfindung, ein gattungsgemäßes Instrument so auszugestalten, daß die beiden genannten Forderungen erfüllt werden können.

Diese Aufgabe wird bei einem chirurgischen Instrument der eingangs beschriebenen Art erfindungsgemäß dadurch gelöst, daß beide Branchen mit einer Ummantelung aus einem elektrisch isolierenden Material versehen sind, wobei an einer Branche ein Anschluß für eine elektrische Verbindungsleitung und an beiden Branchen an deren freiem Ende eine Gewebeanlagefläche freibleibt, daß im Bereich der Lagerstelle zumindest auf der den Gewebeanlageflächen abgewandten Seite die elektrischen Kontaktstellen bei allen Relativstellungen der Branchen nach außen hin abgedeckt sind und daß die elektrischen Kontaktstellen geringfügig über die umgebende Ummantelung hervorstehen.

Die Gesamtummantelung des Instrumentes verhindert eine elektrische Verbindung zwischen dem Instrument und dem umgebenden Gewebe an Stellen, an denen ein solcher Kontakt nicht erwünscht ist. Ein Kontakt kann nur an den Kontaktstellen im Bereich der Lagerstelle auftreten, und an dieser Stelle sind die beiden Branchen elektrisch miteinander verbunden, so daß trotz des Anschlusses an nur einer Branche beide Branchen als Elektroden wirken können. Die elektrische Verbindung ist dabei so ausgestaltet, daß sie nach außen hin nicht zugänglich ist, d. h. die Kontaktstellen werden von den Branchen vollständig abgedeckt und zwar bei beliebigen Winkelstellungen der Branchen, die diese im Betrieb einnehmen können. Um die die Kontaktstellen umgebende Ummantelung gegen Abrieb zu schützen und immer einen zuverlässigen elektrischen Kontakt im Bereich der Kontaktstellen zu gewährleisten, stehen die elektrischen Kontaktstellen geringfügig über die Ummantelung hervor, die Branchen stützen sich also gegeneinander immer im Bereich der Kontaktstellen ab und nicht im Bereich der Ummantelung.

Günstig ist es, wenn die elektrischen Kontaktstellen flächig ausgebildet sind und zumindest an den Kanten der Branchen an die Ummantelung angrenzen, die sich zumindest geringfügig bis auf die Innenseiten der Branchen erstreckt. Die Kontaktstellen garantieren aufgrund ihrer flächigen Ausbildung geringe Stromdichten, im wesentlichen der gesamte Überdeckungsbereich der beiden Branchen in der Umgebung der Lagerstelle steht als Kontaktstelle zur Verfügung. Trotzdem ergibt sich an den Kanten nicht die Gefahr eines unerwünschten elektrischen Kontaktes mit diesen relativ großen flächigen Kontaktstellen, da die Ummantelung an den Kanten bis auf die Innenseiten der Branchen herumgezogen ist und somit nach außen hin die Kontaktstellen schützt.

Besonders vorteilhaft ist es, wenn die elektrischen Kontaktstellen allseits von der Ummantelung umgeben und bei jeder Relativstellung der Branchen vollständig von den Branchen abgedeckt sind. Dies ist nicht bei allen Instrumenten möglich, bei scherenartigen Instrumenten kann es auch vorgesehen sein, daß die flächigen Kontaktstellen unmittelbar in die innenseitigen Gewebeanlageflächen übergehen.

Bei einer bevorzugten Ausführungsform ist vorgesehen, daß die Gewebeanlageflächen an der Innenseite der Branchen längs der Kanten der Branchen an die Ummantelung angrenzen. Auch dadurch wird eine besonders große Gewebeanlagefläche gewährleistet, die zu geringen Stromdichten führt, im Bereich der Kanten ergibt sich aber ein sicherer Schutz gegen unbeabsichtigte Berührung. Günstig ist es, wenn sich die Ummantelung zumindest geringfügig bis auf die Innenseiten der Branchen erstreckt, dies verbessert den Schutz der Gewebeanlageflächen gegen unerwünschte Berührung.

Die Gewebeanlageflächen können bei einer bevorzugten Ausführungsform an der Innenseite der Branchen geringfügig über die angrenzende Ummantelung hervorstehen, so daß auch hier sichergestellt ist, daß die beiden Branchen im Bereich der Gewebeanlageflächen aneinander anliegen und nicht im Bereich der angrenzenden Ummantelung. Dies schützt die Ummantelung vor Abnutzung und Beschädigung.

Günstig ist es, wenn die Ummantelung an Kanten von Gewebeanlageflächen, die als Schneiden wirken, gegenüber diesen Kanten im Schneidbereich geringfügig zurückgesetzt ist, so daß die Kanten der beiden Branchen im Schneidbereich metallisch aneinander entlanggleiten.

Bei einer bevorzugten Ausführungsform ist vorgesehen, daß die Ummantelung im Bereich der Lagerstelle an der Außenseite der Branchen in der Verlängerung der Drehachse eine Durchbrechung aufweist und daß die Durchbrechungen durch Abdeckkappen aus elektrisch isolierendem Material verschlossen sind.

Insbesondere können die Abdeckkappen durch die Köpfe von pilzförmigen Stiften gebildet werden, deren Schäfte in eine hohle Lagerwelle eintauchen.

Auf diese Weise ist es möglich, die mit einer Ummantelung versehenen Branchen miteinander gelenkig zu verbinden oder wieder voneinander zu lösen, ohne daß die Ummantelung in diesem Bereich beschädigt werden muß. Trotzdem ergibt sich im Bereich der Durchbrechungen eine elektrische Abdeckung durch die Abdeckkappen.

Bei einer bevorzugten Ausführungsform eines Instrumentes kann vorgesehen sein, daß die Spitzen der Branchen aus der Ummantelung hervorstehen.

Die nachfolgende Beschreibung bevorzugter Ausführungsformen der Erfindung dient im Zusammenhang mit der Zeichnung der näheren Erläuterung. Es zeigen:
- Figur 1:: ein scherenartiges Instrument mit ummantelten Branchen;
- Figur 2:: eine vergrößerte Ansicht des vorderen Bereichs des Instrumentes der Figur 1;
- Figur 3:: eine Seitenansicht des Instruments der Figuren 1 und 2 in geschlossenem Zustand;
- Figur 4:: eine Schnittansicht längs Linie 4-4 in Figur 2;
- Figur 5:: eine Seitenansicht eines zangenartigen Instrumentes mit ummantelten Branchen;
- Figur 6:: eine Seitenansicht einer Branche des Instruments der Figur 5 von dessen Innenseite her;
- Figur 7:: eine Ansicht des Lagerbereiches des Instruments der Figur 5 in Richtung des Pfeiles A in Figur 6 und
- Figur 8:: eine Schnittansicht längs Linie 8-8 in Figur 5.

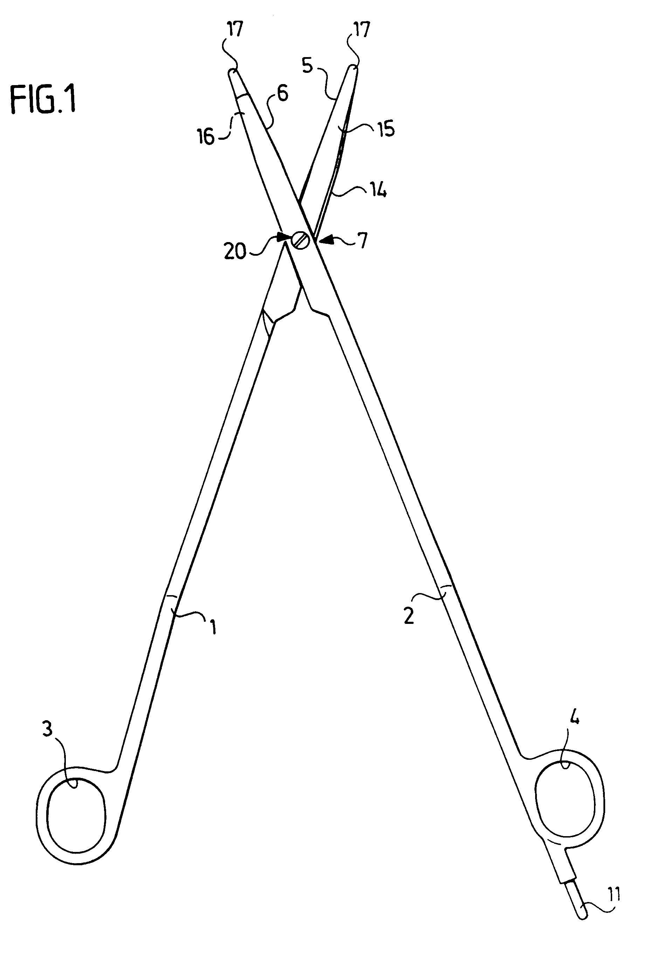

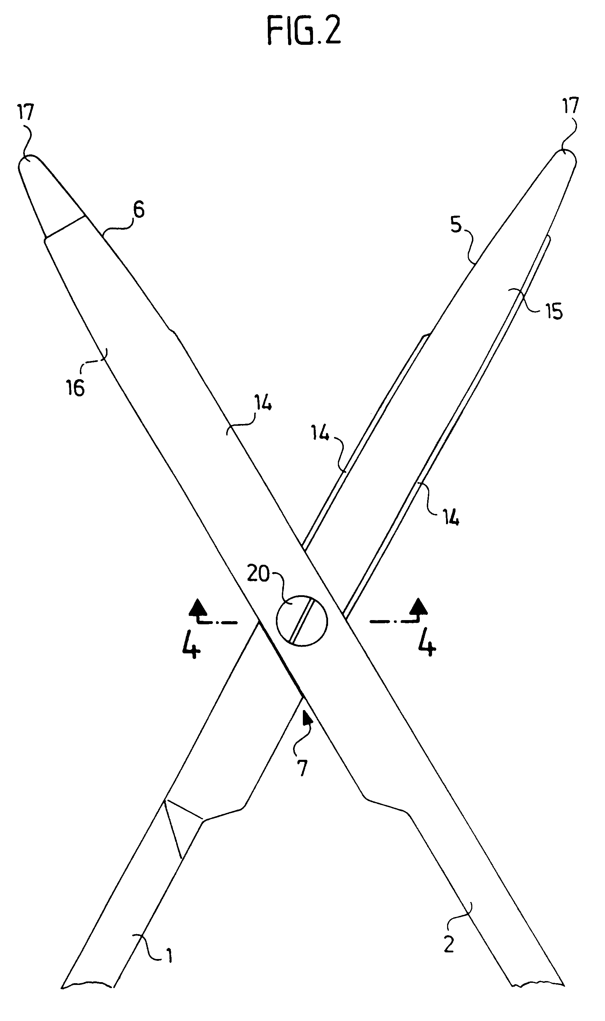

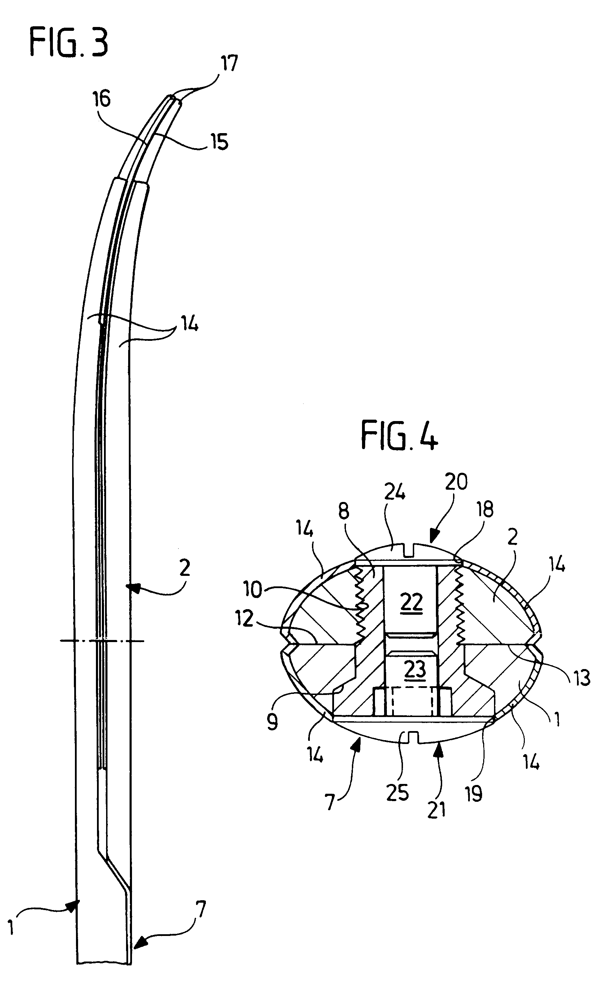

Das in den Figuren 1 bis 4 dargestellte Instrument bildet eine Schere. Es umfaßt zwei Branchen 1, 2 mit jeweils einer Fingeröffnung 3, 4 an einem Ende und einander zugewandten, beim Schließen aneinander entlanggleitenden Schneiden 5, 6 am anderen Ende. Beide Branchen sind an einer Lagerstelle 7 schwenkbar miteinander verbunden. Die Lagerung in diesem Bereich erfolgt durch eine hohle Lagerschraube 8, die eine stufige Bohrung 9 in einer Branche durchsetzt und in eine Innengewindebohrung 10 der anderen Branche eingeschraubt ist (Figur 4).

Die Branchen sind aus Metall gefertigt und bilden gemeinsam eine Elektrode eines Elektrokoagulationsgerätes. Zu diesem Zweck trägt eine der beiden Branchen einen stiftförmigen Anschluß 11, auf den ein Stecker aufgesteckt werden kann, mit dem eine Leitungsverbindung zu einem elektrischen Versorgungsgerät hergestellt wird. Die beiden Branchen 1, 2 liegen im Umgebungsbereich der Lagerstelle 7 flächig aneinander an, die Anlageflächen 12, 13 bilden somit elektrische Kontaktflächen.

Beide Branchen sind bis auf wenige Ausnahmen vollständig von einer elektrisch isolierenden Ummantelung 14 umgeben, so daß ein elektrischer Kontakt mit dem umgebenden Gewebe weitgehend vermieden wird. Nicht ummantelt ist der Stift des Anschlusses 11, nicht ummantelt oder beschichtet sind die als elektrische Kontaktflächen dienenden Anlageflächen 12 und 13, nicht ummantelt sind die an die Schneiden 5 und 6 an der Innenseite der Branchen 1 und 2 anschließenden und sich bis zu den Anlageflächen 12 und 13 erstreckenden und in diese übergehenden Gewebeanlageflächen 15 und 16, und nicht ummantelt sind die aus der Ummantelung 14 hervorstehenden Spitzen 17 der beiden Branchen 1, 2.

Im Bereich der Anlageflächen 12 und 13 erstreckt sich die Ummantelung 14 um die Kanten der Branchen 1, 2 herum bis an die Innenseite der Branchen 1, 2, wobei diese Innenseite im Bereich der Kanten der Branchen 1, 2 nur über eine ganz geringe Breite bedeckt ist, in dem an die Lagerstelle 7 in Richtung auf die Fingeröffnungen 3, 4 verlaufenden Teil jedoch vollständig. Dadurch wird sichergestellt, daß beim Öffnen des Instrumentes in diesem den Fingeröffnungen zugewandten Teil der Branchen keine unerwünschte elektrische Verbindung auftreten kann. Die als Kontaktflächen wirkenden Anlageflächen 12 und 13 werden im übrigen in diesem Bereich vollständig von den beiden Branchen 1, 2 überdeckt, so daß unabhängig von der Winkelstellung der Branchen in diesem Bereich kein elektrischer Kontakt mit umgebendem Gewebe möglich ist.

Die Anlageflächen 12, 13 stehen in Richtung auf die jeweils andere Branche geringfügig über die Ummantelung 14 hervor, sind also in diesem Bereich etwas höher ausgebildet. Dadurch ist sichergestellt, daß die Branchen immer im Bereich dieser Anlageflächen 12, 13 aneinander anliegen und nicht im Bereich der Ummantelung 14. Dies führt auch dazu, daß die Ummantelung 14, die üblicherweise aus Kunststoff besteht, gegen Abrieb geschützt wird.

Die als elektrische Kontaktflächen wirkenden Anlageflächen 12, 13 setzen sich bei dem in den Figuren 1 bis 4 dargestellten Ausführungsbeispiel direkt in die Gewebeanlageflächen 15, 16 fort, die durch die gesamte Innenfläche der Branchen zwischen Lagerstelle 7 und Spitze 17 gebildet wird. Auch bei dieser ist vorgesehen, daß die Ummantelung 14 die Branchen auf der Außenseite vollständig umgibt und zumindest bereichsweise um die Kanten der Branchen 1, 2 herum bis auf die Innenseite reicht. Allerdings ist auch hier vorteilhaft, wenn die Gewebeanlagefläche 15, 16 höhenmäßig gering über die Ummantelung 14 hervorsteht, so daß auch in diesem Bereich die Branchen immer an der metallischen Gewebeanlagefläche 15, 16 aneinander anliegen und die Ummantelung 14 geschont wird. Im Bereich der Schneiden 5, 6 wird die Ummantelung 14 ein wenig zurückgesetzt, so daß diese Schneiden 5, 6 frei von einer Ummantelung sind und unmittelbar metallisch aneinander anliegen können.

Die Ummantelung 14 weist außerdem im Bereich der Lagerstelle 7 in der Verlängerung der Lagerschraube 8 Durchbrechungen 18, 19 auf, die durch pilzförmige Abdeckstifte 20, 21 abgedeckt werden. Diese tauchen mit ihren stiftförmigen Schäften 22, 23 in die hohle Lagerschraube 8 ein und sitzen in dieser entweder im Klemmsitz oder sind in diese eingeschraubt. Die mit den Schäften 22 und 23 verbundenen Köpfe 24 bzw. 25 überdecken die Durchbrechungen 18 bzw. 19 und verschließen diese nach außen, so daß auch in diesem Bereich keine unerwünschte elektrische Verbindung zur Umgebung möglich ist.

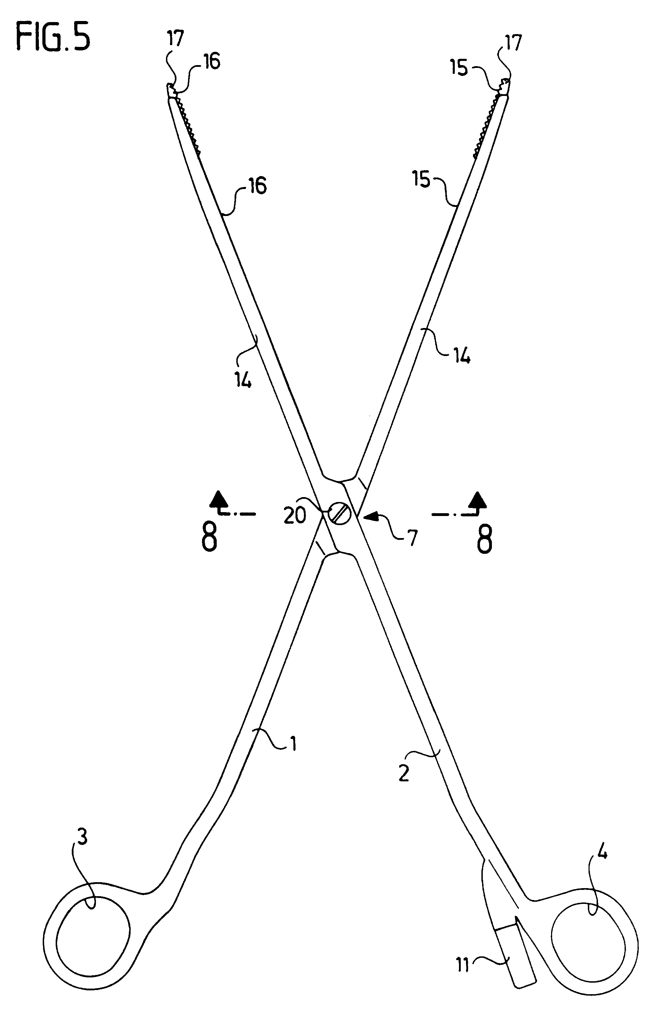

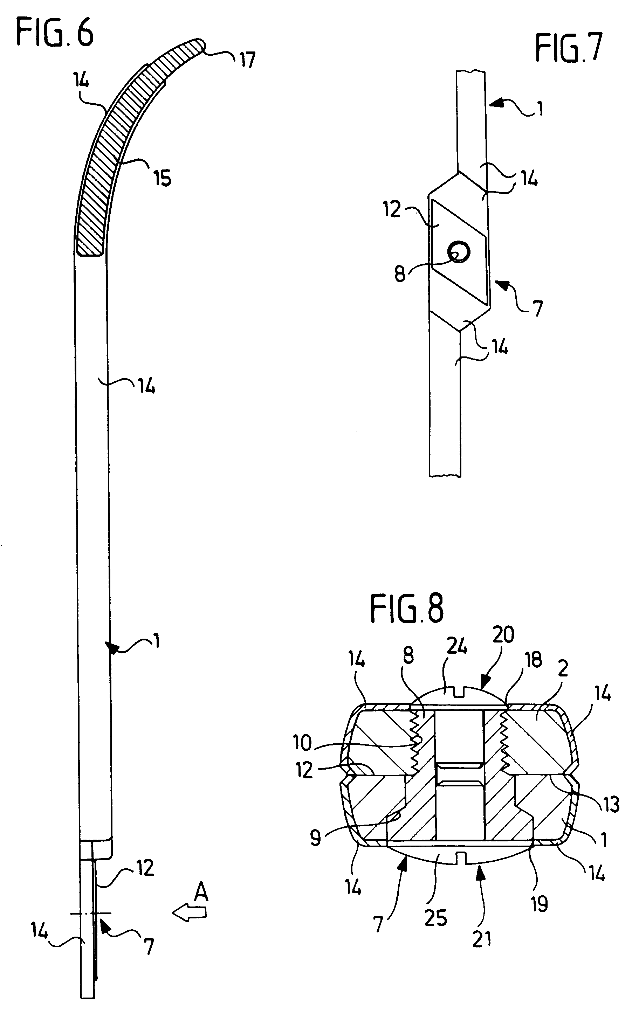

Das in den Figuren 5 bis 8 dargestellte zangenförmige Instrument ist sehr ähnlich aufgebaut wie das scherenförmige Instrument der Figuren 1 bis 4, gleiche Teile werden daher mit denselben Bezugszeichen bezeichnet.

Im Unterschied zu dem Ausführungsbeispiel der Figuren 1 bis 4 gleiten die beiden Branchen beim Schließen hier nicht aneinander vorbei, sondern im Schließzustand liegen sie flächig aneinander an. Dementsprechend befinden sich die Gewebeanlageflächen 15 und 16 nicht in der Verlängerung der den elektrischen Kontakt bewirkenden Anlageflächen 12 und 13 und gehen auch nicht in diese über, sondern diese Gewebeanlageflächen 15 und 16 sind bei diesem Ausführungsbeispiel gegenüber der Ebene der Anlageflächen 12 und 13 um 90° gedreht, sie liegen also jeweils der benachbarten Branche gegenüber.

Dies führt dazu, daß die Anlageflächen 12 und 13 bei diesem Ausführungsbeispiel allseits von der Ummantelung 14 umrandet sind, beim Öffnen des Instrumentes gibt es also im Bereich der Lagerstelle 7 keinerlei Möglichkeit einer elektrischen Verbindung mit der Umgebung.

Auch bei diesem Ausführungsbeispiel sind die Gewebeanlageflächen 15 und 16 gegenüber der umgebenden Ummantelung, die auch hier um die Kanten der Branchen herum bis auf die Innenseite der Branchen gezogen ist, leicht erhöht, so daß auch in diesem Falle beim Schließen des Instrumentes die Gewebeanlageflächen 15 und 16 aneinander zur Anlage kommen und die Ummantelung 14 schonen.

## Patentansprüche

1. Zangen- oder scherenförmiges chirurgisches Instrument mit zwei gegeneinander schwenkbaren Branchen (1, 2), die an einer Lagerstelle (7) drehbar miteinander verbunden sind und in diesem Bereich unter Ausbildung einer elektrischen Kontaktstelle (12, 13) an der Innenseite der Branchen aneinander anliegen, dadurch gekennzeichnet, daß beide Branchen (1, 2) mit einer Ummantelung (14) aus einem elektrisch isolierenden Material versehen sind, wobei an einer Branche (2) ein Anschluß (11) für eine elektrische Verbindungsleitung und an beiden Branchen (1, 2) an deren freiem Ende eine Gewebeanlagefläche (15, 16) freibleibt, daß im Bereich der Lagerstelle (7) zumindest auf der den Gewebeanlageflächen (15, 16) abgewandten Seite die elektrischen Kontaktstellen (12, 13) bei allen Relativstellungen der Branchen (1, 2) nach außen hin abgedeckt sind und daß die elektrischen Kontaktstellen (12, 13) geringfügig über die umgebende Ummantelung (14) hervorstehen.

2. Instrument nach Anspruch 1, dadurch gekennzeichnet, daß die elektrischen Kontaktstellen (12, 13) flächig ausgebildet sind und zumindest an den Kanten der Branchen (1, 2) an die Ummantelung (14) angrenzen, die sich zumindest geringfügig bis auf die Innenseiten der Branchen (1, 2) erstreckt.

3. Instrument nach Anspruch 2, dadurch gekennzeichnet, daß die elektrischen Kontaktstellen (12, 13) allseits von der Ummantelung (14) umgeben und bei jeder Relativstellung der Branchen (1, 2) vollständig von den Branchen (1, 2) abgedeckt sind.

4. Instrument nach einem der voranstehenden Ansprüche, dadurch gekennzeichnet, daß die Gewebeanlageflächen (15, 16) an der Innenseite der Branchen (1, 2) längs der Kanten der Branchen (1, 2) an die Ummantelung (14) angrenzen.

5. Instrument nach Anspruch 4, dadurch gekennzeichnet, daß sich die Ummantelung (14) zumindest geringfügig bis auf die Innenseite der Branchen (1, 2) erstreckt.

6. Instrument nach Anspruch 5, dadurch gekennzeichnet, daß die Gewebeanlageflächen (15, 16) an der Innenseite der Branchen (1, 2) geringfügig über die angrenzende Ummantelung (14) hervorstehen.

7. Instrument nach einem der Ansprüche 4 bis 6, dadurch gekennzeichnet, daß die Ummantelung (14) an Kanten von Gewebeanlageflächen (15, 16), die als Schneiden (5, 6) wirken, gegenüber diesen Kanten im Schneidbereich geringfügig zurückgesetzt ist, so daß die Kanten der beiden Branchen (1, 2) im Schneidbereich metallisch aneinander entlanggleiten.

8. Instrument nach einem der voranstehenden Ansprüche, dadurch gekennzeichnet, daß die Ummantelung (14) im Bereich der Lagerstelle (7) an der Außenseite der Branchen (1, 2) in der Verlängerung der Drehachse eine Durchbrechung (18, 19) aufweist und daß die Durchbrechungen (18, 19) durch Abdeckkappen (20, 21) aus elektrisch isolierendem Material verschlossen sind.

9. Instrument nach Anspruch 8, dadurch gekennzeichnet, daß die Abdeckkappen (20, 21) durch die Köpfe (24, 25) von pilzförmigen Stiften gebildet werden, deren Schäfte (22, 23) in eine hohle Lagerwelle (8) eintauchen.

10. Instrument nach einem der voranstehenden Ansprüche, dadurch gekennzeichnet, daß die Spitzen (17) der Branchen (1, 2) aus der Ummantelung (14) hervorstehen.

## Claims

1. A surgical instrument in the form of forceps or scissors, comprising two mutually pivotable arms (1, 2) which are rotatably interconnected at a pivot point (7) and in this region rest against one another to form an electrical contact point (12, 13) on the inside of the arms, characterised in that both arms (1, 2) are provided with a covering (14) comprising an electrically insulating material, a connection (11) for an electrical connecting line remaining exposed on one arm (2), and a tissue contact surface (15, 16) remaining exposed on both arms (1, 2) at the free end thereof, in that, in the region of the pivot point (7) at least on the side remote from the tissue contact surfaces (15, 16), the electrical contact points (12, 13) are externally covered in all relative positions of the arms (1, 2), and in that the electrical contact points (12, 13) project slightly beyond the surrounding covering (14).

2. An instrument according to claim 1, characterised in that the electrical contact points (12, 13) are flat and adjoin the covering (14) at least at the edges of the arms (1, 2), the covering (14) extending at least slightly onto the insides of the arms (1, 2).

3. An instrument according to claim 2, characterised in that the electrical contact points (12, 13) are surrounded on all sides by the covering (14) and are completely covered by the arms (1, 2) in any relative position thereof.

4. An instrument according to any one of the preceding claims, characterised in that the tissue contact surfaces (15, 16) adjoin the covering (14) on the inside of the arms (1, 2) along the edges thereof.

5. An instrument according to claim 4, characterised in that the covering (14) extends at least slightly onto the inside of the arms (1, 2).

6. An instrument according to claim 5, characterised in that the tissue contact surfaces (15, 16) on the inside of the arms (1, 2) project slightly beyond the adjacent covering (14).

7. An instrument according to any one of claims 4 to 6, characterised in that the covering (14) on edges of tissue contact surfaces (15, 16), which act as blades (5, 6), is slightly set back relative to these edges in the cutting region so that the edges of the two arms (1, 2) slide along one another metallically in the cutting region.

8. An instrument according to any one of the preceding claims, characterised in that the covering (14) has an opening (18, 19) in the region of the pivot point (7) on the outside of the arms (1, 2) along the extent of the rotation axis, and in that the openings (18, 19) are closed by covering caps (20, 21) comprising electrically insulating material.

9. An instrument according to claim 8, characterised in that the covering caps (20, 21) are formed by the heads (24, 25) of mushroom-shaped pins, the shafts (22, 23) of which extend into a hollow bearing shaft (8).

10. An instrument according to any one of the preceding claims, characterised in that the tips (17) of the arms (1, 2) protrude from the covering (14).

## Revendications

1. Instrument chirurgical en forme de pince ou de ciseaux, comprenant deux branches (1, 2) capable de pivoter l'une par rapport à l'autre, lesquelles sont reliées l'une à l'autre en rotation au niveau d'un emplacement de montage (7) et sont en contact l'une contre l'autre dans cette région en formant un emplacement de contact électrique (12, 13) sur le côté intérieur des branches,
caractérisé en ce que les deux branches (1, 2) sont pourvues d'un enrobage (14) en matériau électriquement isolant, une borne (11) étant prévue sur une branche (2) pour une ligne de raccordement électrique, et une surface de contact de tissu (15, 16) restant libre sur les deux branches (1, 2) à leurs extrémités libres, en ce que dans la région de l'emplacement de montage (7) et au moins du côté détourné des surfaces de contact de tissu (15, 16), les emplacements de contact électrique (12, 13) sont recouverts vers l'extérieur dans toutes les positions relatives des branches (1, 2), et en ce que les emplacements de contact électrique (12, 13) dépassent légèrement au-dessus de l'enrobage environnant (14).

2. Instrument selon la revendication 1, caractérisé en ce que les emplacements de contact électrique (12, 13) sont réalisés avec une certaine surface, et sont adjacents à l'enrobage (14) au moins au niveau des arêtes des branches (1, 2), ledit enrobage s'étendant au moins légèrement jusque sur les faces intérieures des branches (1, 2).

3. Instrument selon la revendication 2, caractérisé en ce que les emplacements de contact électrique (12, 13) sont entourés de tous côtés par l'enrobage (14) et sont totalement recouverts par les branches (1, 2) pour chaque position relative des branches (1, 2).

4. Instrument selon l'une des revendications précédentes, caractérisé en ce que les surfaces de contact de tissu (15, 16) sont adjacentes à l'enrobage (14) sur la face intérieure des branches (1, 2) le long des arêtes des branches (1, 2).

5. Instrument selon la revendication 4, caractérisé en ce que l'enrobage (14) s'étend au moins légèrement jusque sur la face intérieure des branches (1, 2).

6. Instrument selon la revendication 5, caractérisé en ce que les surfaces de contact de tissu (15, 16) sur la face intérieure des branches (1, 2) dépassent légèrement au-delà de l'enrobage adjacent (14).

7. Instrument selon l'une des revendications 4 à 6, caractérisé en ce que, au niveau des arêtes des surfaces de contact de tissu (15, 16) qui font office de tranchants (5, 6), l'enrobage (14) est légèrement en retrait dans la région de coupe par rapport aux arêtes de sorte que les arêtes des deux branches (1, 2) coulissent métal sur métal l'une contre l'autre dans la région des tranchants.

8. Instrument selon l'une des revendications précédentes, caractérisé en ce que l'enrobage (14) comporte une traversée (18, 19) dans la région de l'emplacement de montage (7) et sur la face extérieure des branches (1, 2) dans le prolongement de l'axe de rotation, et en ce que les traversées (18, 19) sont obturées par des capuchons de couverture (20, 21) en matériau électriquement isolant.

9. Instrument selon la revendication 8, caractérisé en ce que les capuchons de couverture (20, 21) sont formés par les têtes (24, 25) de tiges en forme de champignon, dont les fûts (22, 23) plongent dans un arbre de montage creux (8).

10. Instrument selon l'une des revendications précédentes, caractérisé en ce que les pointes (17) des branches (1, 2) dépassent hors de l'enrobage (14).
